# EUROPÄISCHE PATENTSCHRIFT

(11) **EP 2 376 392 B1**
(45) Veröffentlichungstag und Bekanntmachung des Hinweises auf die Patenterteilung: **14.11.2012**
(21) Anmeldenummer: 09799519.5
(22) Anmeldetag: 04.12.2009
(51) Int. Cl.: C02F 3/28

(54) **AUFSTROMREAKTOR MIT GESTEUERTER BIOMASSE-RÜCKFÜHRUNG**
UPFLOW REACTOR FEATURING CONTROLLED RECIRCULATION OF BIOMASS
RÉACTEUR À FLUX ASCENDANT À RECIRCULATION DE BIOMASSE COMMANDÉE

(30) Priorität: 10.12.2008 DE 102008061461
(43) Veröffentlichungstag der Anmeldung: 19.10.2011
(73) Patentinhaber: MCB GmbH, 79777 Ühlingen-Birkendorf (DE)
(72) Erfinder: BUCHMÜLLER, Marianne, 79777 Ühlingen-Birkendorf (DE)
(74) Vertreter: Plate, Jürgen
(86) Internationale Anmeldenummer: PCT/EP2009/008663
(87) Internationale Veröffentlichungsnummer: WO 2010/066379

(56) Entgegenhaltungen:
- EP-A1- 1 408 008
- EP-A2- 0 300 348
- DE-A1-102005 050 997

## Beschreibung

Die Erfindung betrifft einen Aufstromreaktor zur biologischen Behandlung von Abwasser. Zur Reinigung von Abwasser wird eine Vielzahl von Verfahren eingesetzt u.a. Filtration und biologische Verfahren, die sich aerober oder anaerober Mikroorganismen bedienen. Insbesondere wird Abwasser, das mit organischen Verunreinigungen in gelöster und ungelöster Form belastet ist, mittels des UASB-Verfahrens (Upflow Anaerobic Sludge Blanket = UASB) behandelt. Das anaerobe Schlammbett (Anaerobic Sludge Blanket) eines UASB-Reaktors enthält verschiedene Bakterienspezies, von denen einige die ungelösten organischen Verunreinigungen in wasserlösliche Stoffe - hauptsächlich organische Fettsäuren umsetzen und hydrolysieren. Hieran anschließend werden die gelösten Stoffe durch im Schlammbett enthaltene anaerobe Mikroorganismen in Biogas umgewandelt und so das Abwasser gereinigt. Biogas ist ein Gasgemisch aus den Bestandteilen Methan und Kohlendioxid sowie Schwefelwasserstoff und anderen Spurengasen. Sofern genügend Biomasse für den Abbau der im Wasser enthaltenen Stoffe vorhanden ist, ergibt sich die optimale hydraulische Aufenthaltszeit für das zu reinigende Wasser im Reaktor aus dem Verschmutzungsgrad, ausgedrückt beispielsweise als Chemischer Sauerstoffbedarf (CSB) und dem gewünschten Reinigungsgrad. Es ist bekannt, dass unter günstigen Bedingungen bereits bei hydraulischen Aufenthaltszeiten im Bereich von wenigen Stunden Reinigungsgrade von mehr als 90 % erreichbar sind.

Ein hoher Reinigungsgrad ist jedoch nur dann dauerhaft zu erzielen, wenn es gelingt, eine ausreichend große Menge an Biomasse im Reaktor zu halten bzw. einen Zuwachs an Biomasse zu erreichen. Die Wachstumsrate von anaerober bzw. aerober Biomasse liegt im Bereich von ca. 0,05 · d⁻¹ bis 0,5 · d⁻¹. Es ist sicherzustellen, dass zumindest gleich viel Biomasse im Reaktor neu gebildet wird, wie ständig ausgeschwemmt wird. Im ungünstigen Fall eines hohen hydraulischen Durchflusses mit einer niedrigen CSB-Konzentration kann auch bei geringer Ausschwemmung die im Reaktor enthaltene Biomasse abnehmen, weil die Bildungsrate für neue Biomasse von der Menge an zugeführtem Substrat bzw. dem CSB des Abwassers abhängt. Hieraus ist ersichtlich, dass der effektiven Biomasse-Rückhaltung eine entscheidende Bedeutung in Bezug auf die Leistungsfähigkeit eines biologischen Reaktors, insbesondere eines solchen mit anaerober Biomasse zukommt.

In bekannten Anaerob-Reaktoren treten aufgrund der intensiven Produktion von Biogas hohe Aufströmgeschwindigkeiten von mehreren m/h auf. Sofern die Mikroorganismen nicht durch besondere Kultivierung auf speziellen Trägem fixiert sind, bewirkt die hohe Aufströmgeschwindigkeit eine Selektion von Spezies, die natürliche Aggregate bilden. Dieser Selektionsprozess beruht darauf, dass Spezies die keine Aggregate bilden, leichter sind, daher vermehrt aus dem Reaktor geschwemmt und schließlich von den aggregierenden Spezies verdrängt werden. Dieser Selektionsprozess erstreckt sich über Zeiträume von mehreren Monaten bis einigen Jahren und führt zur Bildung einer speziellen Schlammform, die gemeinhin als granulierter Schlamm oder auch "Pelletschlamm" bezeichnet wird. Die "Schlammpellets" haben eine Sinkgeschwindigkeit im Wasser von 50 bis 150 m/h, wohingegen Schlammflocken mit ca. 1 m/h absinken.

Typischerweise liegen die Schlammpellets als kugel- oder linsenförmiges Granulat mit Korngrößen von ca. 0,5 bis 2 mm vor. Die Schlammpellets bestehen aus einem porösen Kalkgerüst, das im Laufe des Selektionsprozesses gebildet wird. Die Bakterien siedeln dabei nicht nur auf der Oberfläche der Schlammpellets - wie beispielsweise bei einem massiven, geschlossenen Trägergranulat eines Festbett-Reaktors - sondern sind auch auf den inneren Oberflächen des Kalkgerüstes zu finden.

Durch die Tätigkeit der Mikroorganismen wird Biogas gebildet, das einerseits als Gasblasen aufsteigt, andererseits auch an der Biomasse anhaftet. Durch die teilweise Umhüllung der Schlammpellets mit Biogas sinkt das spezifische Gewicht der Schlammpellets unter die Dichte von Wasser und die Schlammpellets steigen auf. Die nach oben treibenden Schlammpellets werden durch entsprechend angeordnete Gashauben eingefangen und geben dort nach und nach das Gas wieder ab. Die Ablösung des Gases vom einzelnen Schlammpellet wird durch den im Reaktor nach oben hin abnehmenden hydrostatischen Druck dadurch verstärkt, dass das Gas kompressibel ist und sich bei abnehmendem äußeren Druck ausdehnt. Die am Schlammpellet anhaftenden Gasblasen werden mit abnehmendem Druck größer. Die aufgrund der Auftriebsbewegung im Wasser einwirkenden Reibungs- und Scherkräfte haben damit eine größere Angriffsfläche und die Ablösung der Gasblase vom Schlammpellet wird begünstigt. Durch das Ablösen des Gases vom einzelnen Schlammpellet steigt das spezifische Gewicht des Schlammpellets wieder an, so dass dieses in den unteren Bereich des Reaktors zurücksinkt, wo der Prozess von Neuem beginnt. Durch die Bildung von Gas und die Ablösung des Gases von den Schlammpellets kommt ein Kreislauf aus Flotation und Sedimentation in Gang.

Für die Umsetzung der organischen Verunreinigungen spielt der Stofftransport bzw. die Diffusion an der Oberfläche der Schlammpellets eine maßgebliche Rolle. Die Stärke des Diffusionsstromes eines bestimmten Stoffes ist proportional zu dessen Konzentrationsgefälle vom Abwasser zu den Mikroorganismen im Schlammpellet. Das Schlammpellet ist teilweise von einer Hülle aus anhaftendem Biogas umgeben. Das Konzentrationsgefälle und die Diffusion sind umgekehrt proportional zur Dicke dieser anhaftenden Gashülle. Der Umsatz von organischen Verbindungen und damit verbunden die Effizienz des Reinigungsverfahrens läßt sich steigern, indem die den Schlammpellets anhaftende Hülle aus Biogas möglichst schnell abgelöst wird. Es ist hinlänglich bekannt, dass die den Schlammpellets anhaftende Gashülle durch hohe Turbulenz, d.h. durch große Geschwindigkeitsgradienten verringert wird. Allerdings ist hierbei zu berücksichtigen, dass zu heftige Umwälzungen im Reaktor und die damit verbundenen mechanischen Scherkräfte den Wachstumsprozess der Schlammpellets nachhaltig stören oder verhindern können. Im Extremfall kann das fragile Granulat sogar zerstört werden. Dementsprechend ist eine effektive Umwälzung bzw. Kreislaufführung der Biomasse mit schonender Gasabscheidung erstrebenswert.

DE 10 2005 O50 997 A1 offenbart ein Verfahren und einen Reaktor zur Reinigung von Abwasser, das mit organischen Verunreinigungen belastet ist, mittels eines anaeroben Aufström-Schlammbettes (Upflow Anaerobic Sludge Blanket = UASB). Als Schlamm oder Schlammpelletschlämmung vorliegende Biomasse wird in einem Kreislauf geführt, wobei der Anteil von rückgeführter zu gesamter Biomasse im Reaktor pro Tag größer 0,1 · d-1, insbesondere größer 2 · d-1 und besonders bevorzugt größer 10 · d-1 ist. Der Reaktor umfaßt einen Reaktortank, Leitungen, einen Abwassermischer, einen ersten und mindestens einen weiteren Flotationsabscheider zur Trennung von Reaktorwasser, Biomasse und Biogas, einen oder mehrere Mischer zur Vermischung von Biomasse und Biogas und einen Gasseparator zur Trennung von Biomasse und Biogas.

EP 0 170 332 A1 offenbart ein Verfahren und eine Vorrichtung zur anaeroben Behandlung von Abwasser mittels UASB, bei dem ein Behälter verwendet wird, in dessen unteren Bereich das zu reinigende Abwasser geleitet und aus dessen oberen Bereich das gereinigte Abwasser abgeleitet wird. Im Behälter sind anaerobe Mikroorganismen tätig. Zwischen dem Abwassereinlass und dem Überlauf für das gereinigte Abwasser befinden sich im Behälter übereinandergestapelte Gassammler in Form von Hauben, deren oberer Bereich über eine Leitung mit einer Gas-Schlamm-Trenneinrichtung verbunden ist. Durch die Tätigkeit der Mikroorganismen wird Gas erzeugt, das sich am Schlamm anlagert, so dass dieser als sogenannter Schwimmschlamm nach oben aufschwimmt. Dieser Schwimmschlamm wird durch die Haube eingefangen und gibt nach und nach sein Gas wieder ab, so dass er wieder schwerer wird und als sogenannter Sinkschlamm zurück auf den Boden sinkt. Das von den Schlammpellets abgegebene Gas steigt zusammen mit den von den Hauben eingefangenen, freien Gasblasen weiter in den Leitungen nach oben und reißt dabei Schwimmschlammpartikel und Flüssigkeit mit, die in der Gas-Schlamm-Trennkammer abgetrennt werden. Das Gas wird zweckmäßigerweise abgeführt, während die mitgerissene Flüssigkeit, die auch Schlammpartikel enthalten kann, in eine Falleitung gelangt, die zurück auf den Boden des Behälters führt. Dadurch soll der Sinkschlamm am Boden aufgewirbelt werden, was zu einer Auflockerung der Schlammzone im Bodenbereich und einer besseren Vermischung der Mikroorganismen mit dem ankommenden Abwasser führen soll. Da Wasser jedoch relativ schwer ist, ist die Menge des durch das aufschwimmende Gas transportierbaren Abwassers und somit auch die Verwirbelungsleistung des rückgeführten Abwassers begrenzt. Weiterhin ist bekannt, dass Abwasserreaktoren dieser Art über Reaktorhöhen von mindestens 11 m verfügen müssen, bevor der dort beschriebene Effekt eintritt.

EP 0 244 029 A1 beschreibt einen UASB-Reaktor der mit einer Vorrichtung zur Trennung der drei Phasen Wasser, Schlamm und Biogas ausgestattet ist. Die Trennvorrichtung umfaßt Gashauben, die über Durchtrittsöffnungen mit einem Gassammelkasten verbunden sind, wobei die Durchtrittsöffnungen im oberen Bereich der Gashauben unterhalb des Haubenfirstes angeordnet sind. Zusätzlich ist jede Gashaube im Inneren mit Rückhaltekästen ausgerüstet. Die Rückhaltekästen und die Durchtrittsöffnung sind so gestaltet, dass sich ein Gaspolster ausbildet, welches als Barriere für Wasser und Schlamm fungiert. Gaspolster ausbildet, welches als Barriere für Wasser und Schlamm fungiert.

WO 99/51532 lehrt ein Verfahren und eine Vorrichtung zur anaeroben Reinigung von Abwasser in einem Abwasser und Schlamm aufnehmenden Behälter unter Gasentwicklung. Das sich entwickelnde Gas wird durch einen Gassammler aufgefangen und der durch das aufsteigende Gas angetriebene Kreislauf wird zum Auflockern des auf den Boden des Behälters abgesunkenen Sinkschlamms verwendet. Durch einen Gashebeeffekt des aufsteigenden Gases wird der Sinkschlamm vom Boden abgesaugt und getrennt vom Abwasser in den oberen Bereich des Behälters und zurück in das Abwasser geleitet.

EP 0 711 732 A2 beschreibt ein Modul für einen Reaktor zur anaeroben Reinigung von Abwasser, enthaltend eine den Wasserstand im Modul festlegende obere Überlaufschwelle für das gereinigte Abwasser, mehrere über den ganzen Modulquerschnitt gestaffelt angeordnete Auffanghauben für Biogas mit einer Ableitung in einen Gassammelraum und eine obere Abzugsleitung für die von den Abzugshauben nicht erfasste Abluft. Oberhalb des jeweiligen Flotationsabscheiders wird das Biogas in einen Gassammelraum geführt. Die Entnahme des Biogases aus den Auffanghauben erfolgt über eine kurze Rohrleitung.

Untersuchungen haben gezeigt, dass lediglich 10 bis 20 % der in einem Reaktor vorhandenen Biomasse aktiv am Reinigungsprozess teilnimmt. 80 bis 90 % der vorhandenen Biomasse liefert praktisch keinen Beitrag zur Reinigung des Abwassers. Dementsprechend kommt der Rückführung von Biomasse sowie der schonenden Abscheidung von Biogas eine entscheidende Bedeutung für die Effizienz biologischer Verfahren, wie beispielsweise dem UASB-Verfahren zu.

Die bekannten biologischen Reaktoren weisen eine festgelegte Geometrie auf. Nach einer Anlaufzeit von mehreren Wochen bis Monaten stellen sich im Reaktor stabile Betriebsbedingungen ein, wobei die Betriebsparameter innerhalb verfahrensspezifischer Prozessfenster schwanken. Der Begriff "Prozessfenster" bezeichnet hierbei zusammenhängende Bereiche des mehrdimensionalen Raums der Betriebsparameter, zu denen beispielsweise der Gehalt und die Rückführrate der Biomasse im Reaktor, die Zuflußmenge, der chemische Sauerstoffbedarf (CSB), die Temperatur und der pH-Wert des zugeführten Abwassers zählen. Die Prozessfenster sind weitgehend durch die Art der eingesetzten Biomasse, das zugeführte Abwasser und die Reaktorparameter bestimmt. Insbesondere die für die Reaktoreffizienz wichtige Biomasse-Rückführung kann praktisch nicht gesteuert werden.

Die Aufgabe der vorliegenden Erfindung besteht darin, einen Reaktor zur biologischen Behandlung von Abwasser mit erhöhter Effizienz bereitzustellen.

Erfindungsgemäß wird die voranstehende Aufgabe durch einen Aufstromreaktor mit den Merkmalen des Patentanspruchs 1 gelöst.

Die Gashauben des ersten Flotationsabscheiders weisen eine Auslaßöffnung mit steuerbarer Querschnittsfläche auf.

Vorteilhafte Weiterbildungen der Erfindung sind dadurch gekennzeichnet, dass:
- der Aufstromreaktor mindestens einen weiteren bzw. zweiten Flotationsabscheider aufweist, der oberhalb des ersten Flotationsabscheiders angeordnet ist und eine oder mehrere mit der Sammeleinrichtung verbundene Gashauben umfaßt;
- die Gashauben aüch des zweiten Flotationsabscheiders eine Auslaßöffnung mit steuerbarer Querschnittsfläche bzw. variabler Geometrie aufweisen;
- der erste und zweite Flotationsabscheider eine voneinander verschiedene Bauform aufweisen, wobei sich die unterschiedliche Bauform auch aus unterschiedlich gewählten oder einzustellenden Geometrien des ersten und zweiten Flotationsabscheiders ergeben können;
- mindestens ein Randbereich der Auslaßöffnungen von einer verschiebbaren Blende begrenzt ist;
- der Aufstromreaktor Stellglieder zur Betätigung der verschiebbaren Blenden umfasst, wobei die Stellglieder vorzugsweise mit einem hydraulischem Antrieb ausgestattet sind;
- mindestens ein Randbereich der Auslaßöffnung von einer Schlauchblende begrenzt ist;
- die Schlauchblende mit einer Druckeinrichtung für ein Fluid, vorzugsweise für Wasser, verbunden ist;
- die Auslaßöffnung mit einem Siphon ausgestattet ist;
- der Siphon eine haubenartige Form aufweist, wobei der Siphon und die Gashaube vorzugsweise einstückig ausgebildet sind;
- der Siphon rohrförmig ausgebildet ist;
- der Aufstromreaktor eine elektronische Steuerung umfasst;
- der Aufstromreaktor anaerobe und/oder aerobe Biomasse enthält.

Die Erfindung wird im Folgenden anhand von Zeichnungen näher erläutert; es zeigen:
Fig. 1 eine schematische Schnittansicht eines Reaktors mit Biomasse-Rückführung;
Fig. 2 Anordnung der Gashauben eines ersten und eines zweiten Flotationsabscheiders;
Fig. 3a - b eine Gashaube mit Siphon, Auslaßöffnung und stellbarer Blende in perspektivischer Ansicht;
Fig. 4a - b eine Auslaßöffnung mit Schlauchblende; und
Fig. 5 eine elektronische Steuerung für die verschiebbaren Blenden.

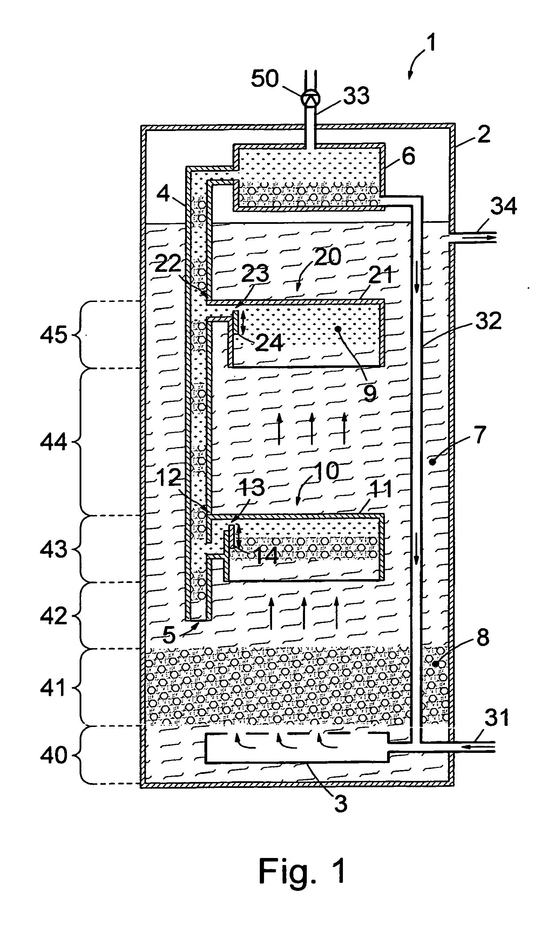

Fig. 1 zeigt schematisch ein Ausführungsbeispiel eines erfindungsgemäßen Aufstromreaktors 1, umfassend einen Reaktortank 2, Leitungen 31 bis 34, einen Abwasserverteiler 3, einen ersten und einen zweiten Flotationsabscheider 10, 20 zur Trennung von Reaktorwasser 7, Biomasse 8 und Biogas 9, eine Sammeleinrichtung 4 und einen Gasseparator 6 zur Trennung von Biomasse 8 und Biogas 9. Der zweite Flotationsabscheider 20 ist vertikal über dem ersten Flotationsabscheider 10 angeordnet. Jeder Flotationsabscheider 10, 20 weist mindestens eine Gashaube 11, 21 auf, die mit der Sammeleinrichtung 4 verbunden ist. Über die Leitung 31 wird dem Aufstromreaktor 1 Abwasser zugeführt und von dem in einer Bodenzone 40 angeordneten Abwasserverteiler 3 unter leichter Wirbelbildung in eine Fermentationszone 41 abgegeben. Gereinigtes Abwasser wird über die im oberen Bereich des Aufstromreaktors angeordnete Leitung 34 abgeführt. In der Fermentationszone 41 befindet sich die Biomasse 8 als Bett aus Schlamm oder Granulatschlämmung. Die in der Biomasse 8 enthaltenen Bakterien bauen die organischen Inhaltsstoffe des Abwassers ab, wobei Biogas 9 gebildet wird. In der Fermentationszone 41 geht das Biogas 9 teilweise in Lösung in das Reaktorwasser 7 über, zum anderen Teil bildet es feine Bläschen die an der Biomasse 8 haften oder frei im Reaktorwasser 7 aufsteigen. Die an der Bildung von Biogas 9 beteiligte Biomasse 8 wird durch das anhaftende Biogas 9 leichter als das Reaktorwasser 7 und steigt ebenso wie die freien Gasbläschen aus der Fermentationszone 41 in eine erste Driftzone 42 auf. Das von der Biomasse 8 erzeugte Biogas 9 verursacht zusammen mit dem über die Leitung 31 zugeführten Abwasser eine aufwärts gerichtete Strömung sowohl von Biomasse 8 wie auch von Reaktorwasser 7.

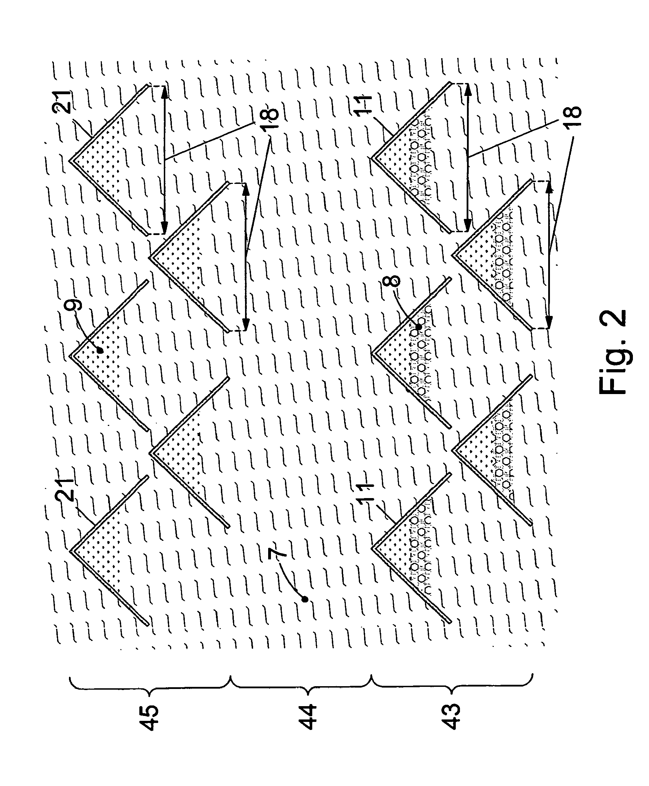

Oberhalb der ersten Driftzone 42 ist der erste Flotationsabscheider 10 mit einer oder mehreren Gashauben 11 (siehe Fig. 2) angeordnet. Die freien Bläschen aus Biogas 9 fangen sich in den Gashauben 11 und bilden ein Gaspolster. Direkt unterhalb des Gaspolsters bildet sich eine Flotationsschicht bestehend aus mit Biogas 9 durchsetzter Biomasse 8.

Jede Gashaube 11 weist in einem oberen Bereich eine Auslaßöffnung 13 auf, deren Weite bzw. Querschnitt mittels einer oder mehrerer Blenden 14 variiert werden kann. Die Auslaßöffnung 13 mündet in einen Siphon 12. Der Siphon 12 ist vorzugsweise hauben- oder rohrförmig ausgebildet und mündet in die Sammeleinrichtung 4. Durch die Auslaßöffnung 13 und den Siphon 12 strömen Biogas 9 und Biomasse 8 aus der Gashaube 11 in die Sammeleinrichtung 4. Aufgrund der Umlenkung der Strömung in dem Siphon 12 werden Biomasse 8 und Biogas 9 intensiv vermengt.

Vorzugsweise ist die Sammeleinrichtung 4 als vertikal verlaufende kasten- oder rohrförmige Steigleitung ausgebildet, die in einem unteren Bereich über eine Öffnung 5 mit dem Innenraum des Aufstromreaktors 1 in Verbindung steht. Durch die Öffnung 5 gelangt Reaktorwasser 7 in die Sammeleinrichtung 4. Das von den Gashauben 11 in die Sammeleinrichtung 4 strömende Gemenge aus Biogas 9 und Biomasse 8 vermischt sich mit dem in der Sammeleinrichtung 4 befindlichen Reaktorwasser 7 und bildet ein Gemisch, dessen Dichte wesentlich geringer ist als die Dichte des Reaktorwassers 7. Aufgrund des Dichteunterschieds steigt das Gemisch in der Sammeleinrichtung 4 nach oben. Die Förderung des Gemisches aus Reaktorwasser 7, Biomasse 8 und Biogas 9 in der Sammeleinrichtung 4 beruht somit auf dem bekannten Mammutpumpen-Prinzip.

In einer alternativen Ausführungsform der Erfindung ist die Sammeleinrichtung 4 in einem unteren Bereich, vorzugsweise unmittelbar unterhalb der Auslaßöffnung 13, geschlossen und steht mit dem Innenraum des Aufstromreaktors 1 nicht in direkter Verbindung. Bei dieser Ausführungsform beruht der Mammutpumpen-Effekt auf dem Dichteunterschied zwischen Biomasse 9 und einem Gemenge aus Biomasse 9 und Biogas 8.

Der Großteil der mit Biogas 9 durchsetzten flotierenden Biomasse 8 wird im Flotationsabscheider 10 aufgefangen und über die Sammeleinrichtung 4 zum Gasseparator 6 gefördert. Reaktorwasser 7, das die Gashauben 11 des Flotationsabscheiders 10 umströmt und in eine zweite Driftzone 44 gelangt, führt nur eine geringe Menge flotierender Biomasse 8 mit. In der Driftzone 44 nimmt der hydrostatische Druck stetig bis auf etwa 1 atm ab. Hierdurch bildet das der flotierenden Biomasse 8 anhaftende Biogas 9 zunehmend größere Blasen, die sich schließlich ablösen. Durch die Ablösung des Biogases 9 nimmt das spezifische Gewicht der Biomasse 8 wieder zu, so dass diese auf den Boden des Reaktors zurücksinkt. Die Abschirmung durch den ersten Flotationsabscheider 10 (siehe Fig. 2) in Verbindung mit der erhöhten Gasabgabe in der Driftzone 44 bewirkt, dass das Reaktorwasser 7, das an die Oberfläche der Wassersäule im Reaktor gelangt und über die Leitung 34 abgeführt wird, praktisch frei von Biomasse 8 ist. Das in den Gashauben 21 des zweiten Flotationsabscheiders 20 gesammelte Biogas 9 strömt durch Auslaßöffnungen 23 in die Sammeleinrichtung 4. In einer vorteilhaften Weiterbildung der Erfindung sind eine oder mehrere der Gashauben 21 jeweils mit einer stellbaren Blende 24 und einem Siphon 22 ausgestattet.

Im Gasseparator 6 wird das Gemenge aus Biomasse 8 und Biogas 9 getrennt, wobei die Biomasse 8 unter Einwirkung der Schwerkraft über die Leitung 32 in den Bodenbereich des Aufstromreaktors zurückfließt. Vorzugsweise ist die Leitung 32 mit der Leitung 31 verbunden, so dass die rückgeführte Biomasse 8 mit dem zugeführten Abwasser vermischt wird.

Das im Gasseparator 6 freigesetzte Biogas 9 wird über eine Leitung 33 abgeführt. Vorzugsweise ist der Volumenstrom des über die Leitung 33 abgeführten Biogases 9 über ein Durchflußregelventil 50 in der Leitung 33 steuerbar. Über den Volumenstrom des abgeführten Biogases 9 wird der Druck im Gasseparator 6 und damit verbunden in der Sammeleinrichtung 4 und den Gashauben 11, 21 geregelt. Somit kann mittels des Durchflußregelventils 50 die Dicke der Gaspolster in den Gashauben 11, 21 gesteuert werden.

Fig. 2 zeigt einen Schnitt quer zur Längsachse der Flotationsabscheider 10, 20. Die Gashauben 11, 21 sind vorzugsweise als Hohlkörper mit einer polygonalen oder halbkreisartigen Hüllwand, insbesondere in Form eines umgekehrten V oder umgekehrten U mit einer nach unten weisenden Öffnung 18 ausgebildet. Wie in Fig. 2 dargestellt, sind die Gashauben 11, 21 in jedem der Flotationsabscheider 10, 20 in zwei oder mehreren übereinander liegenden horizontalen Ebenen angeordnet. In einer Ebene sind die Gashauben 11, 21 jeweils parallel und voneinander beabstandet angeordnet. Durch die Spalte zwischen benachbarten Gashauben 11, 21 tritt aufsteigendes Reaktorwasser 7 hindurch und strömt nach oben. In jedem der Flotationsabscheider 10, 20 sind die Reihen der Gashauben 11, 21 in übereinander liegenden Ebenen derart zueinander versetzt, dass die vertikalen Projektionen der Öffnungen 18 der Gashauben 11, 21 eine geschlossene Fläche bilden, die den Innenquerschnitt des Reaktortanks 2 teilweise oder vollständig abdeckt. Durch diese labyrinthartige Anordnung der Gashauben 11, 21 werden Biomasse 8 und Biogas 9 nahezu vollständig aufgefangen.

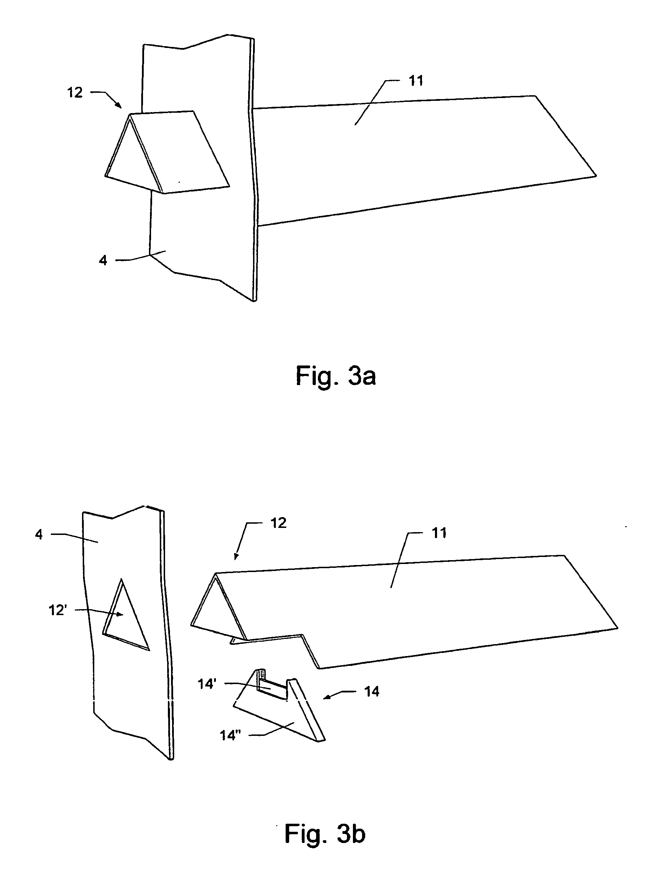

Fig. 3a und 3b zeigen in perspektivischer Ansicht bzw. in Explosionsdarstellung ein Ausführungsbeispiel einer Gashaube 11 mit Siphon 12, Auslaßöffnung 13 und steuerbarer Blende 14. Die Blende 14 wird mittels eines - in Fig. 3a und 3b nicht gezeigten - Stellgliedes verfahren. Das Stellglied ist vorzugsweise mit einem hydraulischen Antrieb ausgestattet, wobei sich Wasser als Hydraulikfluid besonders eignet. Vorteilhafterweise umfasst der hydraulische Antrieb einen Zylinder mit verschiebbarem Kolben, wobei der Zylinder über eine Leitung, beispielsweise einen Schlauch mit einer außerhalb des Reaktortanks 2 angeordneten Druckeinrichtung bzw. Pumpe verbunden ist.

Alternativ bzw. ergänzend zu der in den Fig. 3a und 3b gezeigten Ausführungsform mit einer unterhalb der Auslaßöffnung 13 angeordneten Blende 14 kann die Gashaube 11 mit einer oder mehreren, oberhalb und/oder seitlich der Auslaßöffnung 13 angeordneten Blenden ausgestattet sein.

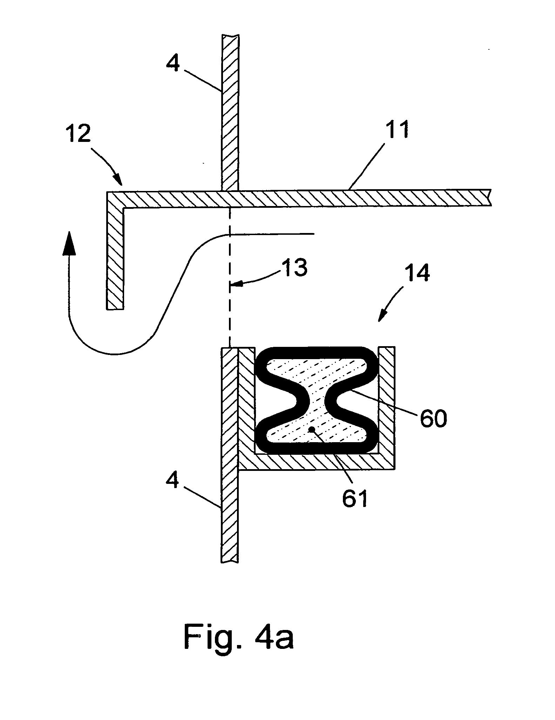

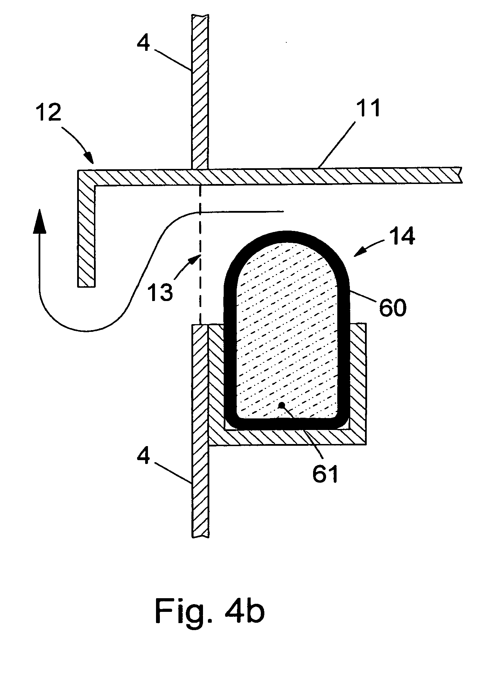

In einer vorteilhaften Weiterbildung der Erfindung sind die stellbaren Blenden 14 als Schlauchblenden ausgebildet. Fig. 4a und 4b zeigen schematisch in Schnittansicht ein Beispiel einer erfindungsgemäßen Schlauchblende 14. Die Schlauchblende 14 umfasst einen fluiddicht abgeschlossen röhrenförmigen Hohlkörper 60 aus einem elastischen Material, wie beispielsweise Gummi. Der Innenraum des Hohlkörpers 60 ist mit einer (in den Fig. 4a und 4b nicht gezeigten) Druckeinrichtung, wie einer Pumpe verbunden. Mittels der Druckeinrichtung wird der elastische Hohlkörper 60 mit einer vorgegebenen Menge eines Fluids 61, bei dem es sich vorzugsweise um Wasser handelt, gefüllt. Je nach der Füllmenge des Fluids 61 ist die Schlauchblende 14 mehr oder minder weit geöffnet.

In einer weiteren Ausführungsform der Erfindung sind die Blenden 14, 24 bzw. die Flotationsabscheider 10, 20 mit mechanischen Stellgliedern, beispielweise einer Linearführung mit Spindelantrieb ausgerüstet, die manuell oder mittels eines Motors betätigt werden. Insbesondere zur manuellen Betätigung sind die mechanischen Stellglieder der Blenden 14 mit Wellen gekoppelt, wobei die Wellen flüssigkeitsdicht durch die Wand des Reaktortanks 2 geführt und somit von außen zugänglich sind.

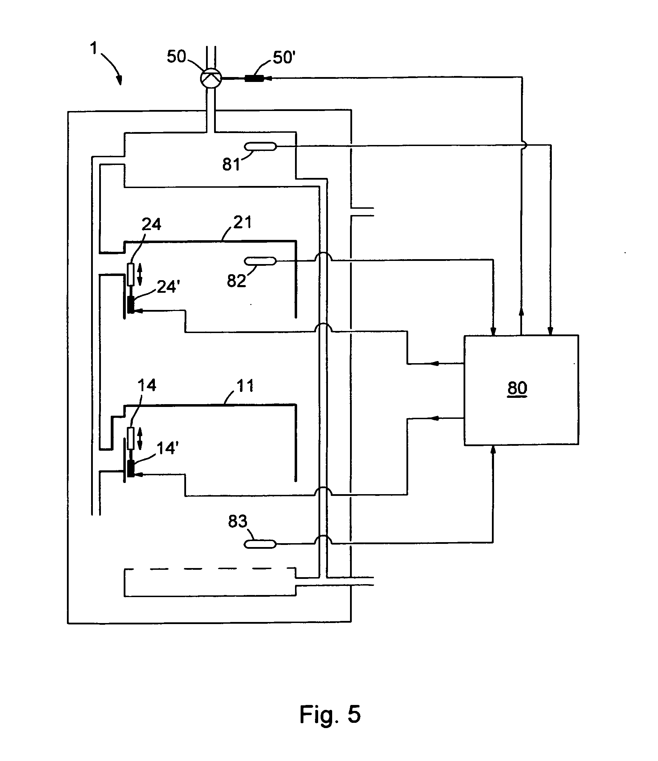

In Fig. 5 ist schematisch ein Ausführungsbeispiel der Erfindung gezeigt, bei welchem der Aufstromreaktor 1 eine elektronische Steuerung 80 und einen oder mehrere Sensoren sowie ein oder mehrere Stellglieder umfasst. Bei den Sensoren handelt es sich beispielsweise um Druck-, Temperatur-, Sauerstoff- oder pH-Fühler, die an verschiedenen Positionen in dem Aufstromreaktor 1 angebracht sein können. Die Sensoren sind in Fig. 5 mit den Bezugszeichen 81, 82, und 83 und die Stellglieder mit 14', 24', 50' gekennzeichnet. Die Stellglieder 14', 24', 50' umfassen im Allgemeinen einen elektrischen oder hydraulischen Antrieb und dienen vorzugsweise zur Anstellung der Blenden 14, 24 und des Durchflußregelventils 50. Die Steuerung 80 weist für jeden der Sensoren 61, 62, 63 einen Signal-Eingang und für jedes der Stellglieder 14', 24', 50' einen Steuer-Ausgang auf. Die Steuerung 80 ist vorteilhaft als Speicher-programmierbare Steuerung (SPS) ausgestaltet. In dem SPS-Speicher ist ein programmierter Algorithmus nach Art eines Regelkreises abgelegt, mit dem die Steuersignale für die Stellglieder 14', 24', 50' in Abhängigkeit von den Signalen der Sensoren 61, 62, 63 berechnet werden.

## Patentansprüche

1. Aufstromreaktor (1) mit Biomasse-Rückführung zur biologischen Behandlung von Abwasser mit mindestens einem ersten Flotationsabscheider (10), der eine oder mehrere mit einer Sammeleinrichtung (4) verbundene Gashauben (11) und eine oder mehrere Vorrichtungen (13, 23) zur Steuerung der Biomasse-Rückführung umfasst, **dadurch gekennzeichnet, dass** die Gashauben (11) jeweils eine Auslaßöffnung (13) mit steuerbarer Querschnittsfläche aufweisen.

2. Aufstromreaktor (1) nach Anspruch 1, **dadurch gekennzeichnet, dass** der Aufstromreaktor (1) einen zweiten Flotationsabscheider (20) aufweist, der oberhalb des ersten Flotationsabscheiders (10) angeordnet ist und eine oder mehrere mit der Sammeleinrichtung (4) verbundene Gashauben (21) umfaßt.

3. Aufstromreaktor (1) nach Anspruch 2, **dadurch gekennzeichnet, dass** die Gashauben (21) jeweils eine Auslaßöffnung (23) mit steuerbarer Querschnittsfläche aufweisen.

4. Aufstromreaktor (1) nach Anspruch 2 oder 3, **dadurch gekennzeichnet, dass** der erste Flotationsabscheider (10) und der zweite Flotationsabscheider (20) eine voneinander verschiedene Bauform aufweisen.

5. Aufstromreaktor (1) nach einem oder mehreren der Ansprüche 1 bis 4, **dadurch gekennzeichnet, dass** mindestens ein Randbereich der Auslaßöffnungen (13, 23) von einer verschiebbaren Blende (14, 24) begrenzt ist.

6. Aufstromreaktor (1) nach Anspruch 5, **dadurch gekennzeichnet, dass** der Aufstromreaktor (1) Stellglieder zur Betätigung der verschiebbaren Blenden (14, 24) umfasst, wobei die Stellglieder vorzugsweise mit einem hydraulischen Antrieb ausgestattet sind.

7. Aufstromreaktor (1) nach einem oder mehreren der Ansprüche 1 bis 4, **dadurch gekennzeichnet, dass** mindestens ein Randbereich der Auslaßöffnungen (13) und ggf. (23) jeweils von einer Schlauchblende begrenzt ist.

8. Aufstromreaktor (1) nach Anspruch 7, **dadurch gekennzeichnet, dass** die Schlauchblende mit einer Druckeinrichtung für ein Fluid, vorzugsweise für Wasser, verbunden ist.

9. Aufstromreaktor (1) nach einem oder mehreren der Ansprüche 1 bis 8, **dadurch gekennzeichnet, dass** die Auslaßöffnungen (13) und ggf. (23) jeweils mit einem Siphon (12) bzw. (22) ausgestattet sind.

10. Aufstromreaktor (1) nach Anspruch 9, **dadurch gekennzeichnet, dass** der Siphon (12, 22) haubenförmig ausgebildet ist.

11. Aufstromreaktor (1) nach Anspruch 10, **dadurch gekennzeichnet, dass** der Siphon (12, 22) und die Gashaube (11, 21) einstückig ausgebildet sind.

12. Aufstromreaktor (1) nach Anspruch 11, **dadurch gekennzeichnet, dass** der Siphon (12, 22) rohrförmig ausgebildet ist.

13. Aufstromreaktor (1) nach einem oder mehreren der Ansprüche 1 bis 12, **dadurch gekennzeichnet, dass** der Aufstromreaktor (1) eine elektronische Steuerung (80) umfasst.

14. Aufstromreaktor (1) nach einem oder mehreren der Ansprüche 1 bis 13, **dadurch gekennzeichnet, dass** der Aufstromreaktor (1) anaerobe und/oder aerobe Biomasse (8) enthält.

## Claims

1. Upflow reactor (1) having recirculation of biomass for the biological treatment of waste water having at least one first flotation separator (10) which comprises one or more gas hoods (11) connected to a collector (4) and one or more devices (13, 23) for controlling the recirculation of the biomass, **characterized in that** the gas hoods (11) in each case comprise an outlet opening (13) having a controllable cross-sectional area.

2. Upflow reactor (1) according to Claim 1, **characterized in that** the upflow reactor (1) comprises a second flotation separator (20) which is arranged above the first flotation separator (10) and comprises one or more gas hoods (21) connected to the collector (4).

3. Upflow reactor (1) according to Claim 2, **characterized in that** the gas hoods (21) in each case comprise an outlet opening (23) having a controllable cross-sectional area.

4. Upflow reactor (1) according to Claim 2 or 3, **characterized in that** the first flotation separator (10) and the second flotation separator (20) are of a type differing from one another.

5. Upflow reactor (1) according to one or more of Claims 1 to 4, **characterized in that** at least one edge region of the outlet openings (13, 23) is restricted by a slideable diaphragm (14, 24).

6. Upflow reactor (1) according to Claim 5, **characterized in that** the upflow reactor (1) comprises actuators for actuating the slideable diaphragms (14, 24), wherein the actuators are preferably equipped with a hydraulic drive.

7. Upflow reactor (1) according to one or more of Claims 1 to 4, **characterized in that** at least one edge region of the outlet openings (13) and optionally (23) is in each case restricted by a flexible-tube diaphragm.

8. Upflow reactor (1) according to Claim 7, **characterized in that** the flexible-tube diaphragm is connected to a pressure appliance for a fluid, preferably for water.

9. Upflow reactor (1) according to one or more of Claims 1 to 8, **characterized in that** the outlet openings (13) and optionally (23) are in each case equipped with a siphon (12) or (22).

10. Upflow reactor (1) according to Claim 9, **characterized in that** the siphon (12, 22) is constructed in a hood-shape.

11. Upflow reactor (1) according to Claim 10, **characterized in that** the siphon (12, 22) and the gas hood (11, 21) are formed so as to be in one piece.

12. Upflow reactor (1) according to Claim 11, **characterized in that** the siphon (12, 22) is formed so as to be tubular.

13. Upflow reactor (1) according to one or more of Claims 1 to 12, **characterized in that** the upflow reactor (1) comprises an electronic control (80).

14. Upflow reactor (1) according to one or more of Claims 1 to 13, **characterized in that** the upflow reactor (1) contains anaerobic and/or aerobic biomass (8).

## Revendications

1. Réacteur à flux ascendant (1) à recirculation de biomasse servant au traitement biologique des eaux usées, comportant au moins un premier séparateur par flottation (10), qui comporte une ou plusieurs calottes à gaz (11) reliées à un système collecteur (4) et un ou plusieurs dispositifs (13, 23) servant à commander la recirculation de biomasse, **caractérisé en ce que** les calottes à gaz (11) présentent respectivement un orifice de sortie (13) doté d'une surface de section transversale pouvant être commandée.

2. Réacteur à flux ascendant (1) selon la revendication 1, **caractérisé en ce que** le réacteur à flux ascendant (1) présente un deuxième séparateur par flottation (20), qui est disposé au-dessus du premier séparateur par flottation (10) et qui comporte une ou plusieurs calottes à gaz (21) reliées au système collecteur (4).

3. Réacteur à flux ascendant (1) selon la revendication 2, **caractérisé en ce que** les calottes à gaz (21) présentent respectivement un orifice de sortie (23) doté d'une surface de section transversale pouvant être commandée.

4. Réacteur à flux ascendant (1) selon la revendication 2 ou 3, **caractérisé en ce que** le premier séparateur à flottation (10) et le deuxième séparateur à flottation (20) présentent une forme de construction différente l'une de l'autre.

5. Réacteur à flux ascendant (1) selon l'une quelconque ou plusieurs des revendications 1 à 4, **caractérisé en ce qu'**au moins une zone de bord des orifices de sortie (13, 23) est délimitée par un obturateur (14, 24) pouvant être coulissé.

6. Réacteur à flux ascendant (1) selon la revendication 5, **caractérisé en ce que** le réacteur à flux ascendant (1) comporte des organes de commande servant à actionner les obturateurs (14, 24) pouvant être coulissés, où les organes de commande sont équipés de préférence d'un mécanisme d'entraînement hydraulique.

7. Réacteur à flux ascendant (1) selon l'une quelconque ou plusieurs des revendications 1 à 4, **caractérisé en ce qu'**au moins une zone de bord des orifices de sortie (13) et éventuellement (23) est délimitée respectivement par un obturateur de tuyau flexible.

8. Réacteur à flux ascendant (1) selon la revendication 7, **caractérisé en ce que** l'obturateur de tuyau flexible est relié à un système de compression pour un fluide, de préférence pour de l'eau.

9. Réacteur à flux ascendant (1) selon l'une quelconque ou plusieurs des revendications 1 à 8, **caractérisé en ce que** les orifices de sortie (13) et éventuellement (23) sont équipés respectivement d'un siphon (12) ou (22).

10. Réacteur à flux ascendant (1) selon la revendication 9, **caractérisé en ce que** le siphon (12, 22) est réalisé de manière à présenter une forme de calotte.

11. Réacteur à flux ascendant (1) selon la revendication 10, **caractérisé en ce que** le siphon (12, 22) et la calotte à gaz (11, 21) sont réalisés d'un seul tenant.

12. Réacteur à flux ascendant (1) selon la revendication 11, **caractérisé en ce que** le siphon (12, 22) est réalisé de manière à présenter une forme tubulaire.

13. Réacteur à flux ascendant (1) selon l'une quelconque ou plusieurs des revendications 1 à 12, **caractérisé en ce que** le réacteur à flux ascendant (1) comporte une commande électronique (80).

14. Réacteur à flux ascendant (1) selon l'une quelconque ou plusieurs des revendications 1 à 13, **caractérisé en ce que** le réacteur à flux ascendant (1) contient de la biomasse (8) anaérobie et/ou aérobie.
